# DEMANDE DE BREVET EUROPEEN

(11) **EP 2 070 511 A2**
(43) Date de publication de la demande: **17.06.2009**
(21) Numéro de dépôt: 08164282.9
(22) Date de dépôt: 12.09.2008
(51) Int. Cl.: A61K 8/19, A61K 8/26, A61K 8/27, A61K 8/44, A61K 8/46, A61K 8/73, A61K 8/81, A61K 8/84, A61K 8/91, A61Q 5/02, A61Q 5/12

(54) **Composition cosmétique comprenant au moins un polymère cationique particulier, au moins un agent tensioactif, au moins un polymère cationique ou amphotère et au moins une particule minérale, et procédé de traitement cosmétique mettant en oeuvre ladite composition**

(30) Priorité: 14.09.2007 FR 0757590
(71) Demandeur: L'Oreal, 75008 Paris (FR)
(72) Inventeur: Fack, Géraldine, 92300 LEVALLOIS PERRET (FR)
(74) Mandataire: Dossmann, Gérard

(57) **Abrégé**

La présente invention concerne une composition comprenant dans un milieu cosmétiquement acceptable :
- (i) un ou plusieurs polymères cationiques qui est obtenu par polymérisation d'un mélange de monomères comprenant un ou plusieurs monomères vinyliques substitués par un ou plusieurs groupes amino, un ou plusieurs monomères vinyliques non ioniques hydrophobes, un ou plusieurs monomères vinyliques associatifs, et un ou plusieurs monomères non ioniques hydroxylés,
- (ii) un ou plusieurs agents tensioactifs,
- (iii) un ou plusieurs polymères cationiques ou amphotères différents des polymères (i), et
- (iv) une ou plusieurs particules d'un ou plusieurs composés minéraux.

La présente invention concerne également un procédé de traitement cosmétique la mettant en oeuvre et son utilisation comme shampoing conditionneur ou après-shampoing.

## Description

La présente invention concerne une composition de traitement cosmétique des matières kératiniques, de préférence humaines, en particulier des fibres kératiniques, et plus particulièrement des cheveux, comprenant (i) un ou plusieurs polymères cationiques particuliers, (ii) un ou plusieurs agents tensioactifs, (iii) un ou plusieurs polymères cationiques ou amphotères différents des polymères (i) et (iv) une ou plusieurs particules minérales.

La présente invention concerne également un procédé de traitement des matières kératiniques mettant en oeuvre ladite composition cosmétique et son utilisation comme shampooing ou après-shampooing.

Pour le nettoyage et/ou le lavage des matières kératiniques telles que les cheveux, l'utilisation de compositions détergentes (telles que les shampooings ou des après-shampooings) à base essentiellement d'agents tensioactifs classiques de type notamment anionique, non ionique et/ou amphotère, mais plus particulièrement de type anionique, est courante. Ces compositions sont appliquées sur des cheveux mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi pour améliorer les propriétés cosmétiques des compositions ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que des permanentes, des teintures ou décolorations), il est maintenant usuel d'introduire dans ces dernières des agents cosmétiques complémentaires dit agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

De tels agents conditionneurs confèrent aux cheveux lavés, secs ou mouillés, une facilité de démêlage et une douceur nettement accrue par rapport à ce qui peut être obtenu avec les compositions nettoyantes qui en sont exemptes. Ces agents sont notamment des polymères cationiques ou amphotères hydrosolubles.

Par ailleurs, il est en outre connu d'ajouter dans ces compositions lavantes des particules telles que des agents de nacrage ou nacrants afin de leur conférer un aspect ou un effet irisé, moiré ou métallisé, des agents insolubles dans le milieu tels que des silicones afin d'améliorer la douceur, le toucher et le démêlage des cheveux ou encore des agents destinés au traitement du cuir chevelu tels que des agents antipelliculaires ou anti-chutes.

Toutefois, ces agents particulaires présentent généralement l'inconvénient d'être difficiles à maintenir en dispersion régulière ou en solution dans le milieu ce qui peut notamment conduire à un alourdissement et un ternissement de la chevelure.

Ainsi, il a été observé que ces compositions peuvent évoluer sensiblement au cours du temps dans des conditions normales de stockage en fonction de la température, notamment au niveau de leur viscosité ainsi que de leur aspect visuel et conférer des propriétés cosmétiques non satisfaisantes aux cheveux. En particulier, ces compositions présentent souvent un aspect visuel trouble ainsi qu'une texture filante à 45°C ou à température ambiante dans des conditions normales de stockage.

De plus, ces compositions ne présentent pas toujours de bonnes propriétés d'usage car elles ne sont pas suffisamment visqueuses pour pouvoir rester un certain temps sur les cheveux sans s'écouler. Ces compositions s'écoulent également facilement entre les doigts au cours de leur utilisation.

Pour remédier à ces inconvénients, différents types de polymères naturels ou synthétiques ont déjà été utilisés dans ces compositions lavantes.

Les polymères naturels, tels que des celluloses, conduisent généralement à des compositions lavantes instables qui présentent un aspect trouble. De plus, ces polymères épaississants présentent l'inconvénient de diminuer la qualité de la mousse ainsi que les performances cosmétiques des shampooings, notamment en rendant les cheveux plus chargés et plus rêches. Par ailleurs, la mousse générée par ces compositions ne se développe pas facilement que ce soit en vitesse ou en abondance et n'est généralement pas suffisamment douce.

Les polymères synthétiques associatifs sont également classiquement utilisés pour stabiliser et épaissir les compositions lavantes, en particulier les polymères associatifs anioniques tels que les polymères acryliques réticulés anioniques du type Carbopol^{®} ou Pemulen^{®}. Néanmoins, ces polymères présentent également l'inconvénient de diminuer les performances cosmétiques des shampooings, notamment en rendant les cheveux plus chargés et plus rêches. Ils peuvent par ailleurs présenter des difficultés d'utilisation pour des pH inférieurs à 6.

Il existe donc un réel besoin de mettre au point des compositions cosmétiques, par exemple, un shampoing conditionneur ou un après-shampoing, à base de particules et d'agents de conditionnement ne présentant pas l'ensemble des inconvénients décrits ci-dessus, c'est-à-dire qui soient stables dans le temps qui présentent de bonnes propriétés d'usage ainsi qu'un aspect esthétique satisfaisant tout en conférant des propriétés cosmétiques satisfaisantes aux matières kératiniques, en particulier aux cheveux et au cuir chevelu, et qui puissent aussi être formulés à des pH inférieurs à 6.

La demanderesse a découvert, de façon surprenante, qu'il était possible de formuler des compositions pour le traitement cosmétique des matières kératiniques, ayant les propriétés recherchées, comprenant un ou plusieurs agents tensioactifs, un ou plusieurs polymères cationiques particuliers tels que définis ci-après, un ou plusieurs polymères cationiques ou amphotères différents des précédents, une ou plusieurs particules minérales.

En effet, il a été constaté que l'utilisation dudit polymère cationique spécifique dans de telles compositions permettait d'améliorer la texture et la stabilité des shampooings et après-shampoings tout en conférant aux matières kératiniques et plus particulièrement aux cheveux, des propriétés cosmétiques améliorées notamment au niveau du démêlage, de la souplesse, de la malléabilité, du gonflant et de la brillance.

En particulier, la stabilité au stockage des compositions selon l'invention est améliorée aussi bien à température ambiante (20-25°C) qu'à 45°C, notamment au niveau de leur aspect visuel et de leur viscosité. Par « stables » au sens de la présente invention, on entend que l'aspect visuel ainsi que la viscosité de ces compositions n'évoluent pas sensiblement au cours du temps dans des conditions normales de stockage, par exemple pendant les 12, de préférence 24 et de manière encore préférée 30 mois à température ambiante ou pendant les 2 mois à 45°C qui suivent leur fabrication.

La texture améliorée permet au produit, une fois déposé, sur les cheveux de rester un certain temps sans s'écouler. Cette texture améliorée voire gélifiée, permet d'utiliser des quantités moindres de produits. De telles compositions présentent également une texture homogène.

Les compositions ainsi obtenues présentent de bonnes propriétés d'usages, c'est-à-dire que ces compositions peuvent s'écouler et se répartir plus facilement sur l'ensemble de la chevelure.

Les compositions obtenues présentent également de bonnes qualités de mousse, notamment en termes de démarrage, d'abondance, de douceur et de consistance.

La présente invention concerne notamment une composition de traitement cosmétique des matières kératiniques, de préférence humaines, en particulier des fibres kératiniques, et plus particulièrement des cheveux, comprenant dans un milieu cosmétiquement acceptable :
- (i) un ou plusieurs polymères cationiques obtenus par polymérisation d'un mélange de monomères comprenant un ou plusieurs monomères vinyliques substitués par un ou plusieurs groupes amino, un
ou plusieurs monomères vinyliques non ioniques hydrophobes, un ou plusieurs monomères vinyliques associatifs, et un ou plusieurs monomères vinyliques non ioniques hydroxylés,
- (ii) un ou plusieurs agents tensioactifs,
- (iii) un ou plusieurs polymères cationiques ou amphotères différents des polymères (i), et
- (iv) une ou plusieurs particules d'un ou plusieurs composés minéraux.

Elle a aussi pour objet un procédé de traitement cosmétique des matières kératiniques, de préférence humaines, en particulier des fibres kératiniques, et plus particulièrement des cheveux, mettant en oeuvre la composition selon l'invention.

Elle concerne également l'utilisation de la composition selon l'invention comme shampoing conditionneur ou après-shampoing.

D'autres objets et caractéristiques, aspects et avantages de l'invention apparaîtront encore plus clairement à la lecture de la description et des exemples qui suivent.

Par « matières kératiniques », on comprend les cheveux, les cils, les sourcils, la peau, les ongles, les muqueuses ou le cuir chevelu et plus particulièrement les cheveux et par« fibres kératiniques », on comprend les cheveux, les cils, les sourcils.

L'une des caractéristiques essentielles de l'invention est la présence d'un polymère cationique (i) qui est obtenu par polymérisation d'un mélange de monomères comprenant un ou plusieurs monomères vinyliques substitués par un ou plusieurs groupes amino, un ou plusieurs monomères vinyliques non ioniques hydrophobes, un ou plusieurs monomères vinyliques associatifs, et un ou plusieurs monomères vinyliques non ioniques hydroxylés.

Les monomères présents au sein du polymère cationique (i) sont différents les uns des autres.

De préférence, les polymères cationiques (i) sont des polymères épaississants. Au sens de la présente invention, on entend par polymère épaississant, un polymère qui introduit à 1% dans une solution aqueuse ou hydroalcoolique à 30 % d'éthanol, et à pH = 7, permet d'atteindre une viscosité d'au moins 100 cps à 25°C et à un taux de cisaillement de 1s⁻¹. Cette viscosité peut être mesurée à l'aide d'un viscosimètre cône/plan (Rhéomètre Haake R600 ou analogue).

De préférence, ces polymères augmentent par leur présence la viscosité des compositions dans lesquelles ils sont introduits d'au moins 50 cps à 25°C et à un taux de cisaillement de 1s⁻¹.

Le ou les polymères cationiques (i) utilisés dans la composition selon l'invention, et leur procédé de fabrication, sont notamment décrits dans la demande internationale WO 2004/024779.

Par monomère vinylique, on entend au sens de la présente invention un monomère comprenant au moins un groupe R₀CH=C(Ro)- , dans lequel chaque R₀ représente indépendamment H, un groupe alkyle en C₁-C₃₀, -COOH, -CO-OR₀', -O-CO-R₀', -CO-NHR₀', ou -CO-NR₀'R₀", R₀' et R₀" représentant un groupe alkyle en C₁-C₃₀.

Ainsi, par exemple, au sens de la présente invention, les (méth)acrylates et les (méth)acrylamides sont des monomères vinyliques.

Les monomères vinyliques substitués par un ou plusieurs groupes amino utilisables pour la préparation du polymère cationique (i) utilisé dans la composition selon l'invention sont des monomères à insaturation éthylénique, basiques et polymérisables. Les groupes amino peuvent dériver de groupes alkyle mono, di- ou polyaminés, ou bien de groupes hétéroaromatiques contenant un atome d'azote. Les groupes amino peuvent être des amines primaires, secondaires ou tertiaires. Ces monomères peuvent être utilisés sous la forme d'amine ou sous la forme de sel.

De préférence, le ou les monomères vinyliques substitués par un
ou plusieurs groupes amino sont choisis parmi :
- les (méth)acrylates de mono(alkyl en C₁-C₄)amino(alkyle en C₁-C₈),
- les (méth)acrylates de di(alkyl en C₁-C₄)amino(alkyle en C₁-C₈), de préférence les (méth)acrylates de di(alkyl en C₁-C₄)alkylamino(alkyle en C₁-C₆),
- les mono(alkyl en C₁-C₄)amino(alkyl en C₁-C₈)(méth)acrylamides,
- les di(alkyl en C₁-C₄)amino(alkyl en C₁-C₈)(méth)acrylamides,
- les (méth)acrylamides à groupement hétérocyclique contenant un atome d'azote,
- les (méth)acrylates à groupement hétérocyclique contenant un atome d'azote,
- les hétérocycles azotés à groupement(s) vinyle(s),
- et leurs mélanges.

A titre de monomères vinyliques substitués par un ou plusieurs groupes amino préférés, on peut citer :
- les (méth)acrylates de mono- ou di-(alkyl en C₁-C₄)amino(alkyle en C₁-C₄), tels que le (méth)acrylate de 2-(N,N-diméthylamino)éthyle, le (méth)acrylate de 3-(N,N-diméthylamino)propyle, le (méth)acrylate de 4-(N,N-diméthylamino)butyle, le (méth)acrylate de (N,N-diméthylamino)-t-butyle, le (méth)acrylate de 2-(N,N-diéthylamino)éthyle, le (méth)acrylate de 3-(N,N-diéthylamino)-propyle, le (méth)acrylate de 4-(N,N-diéthylamino)butyle, le (méth)acrylate de 2-(N,N-dipropylamino)éthyle, le (méth)acrylate de 3-(N,N-dipropylamino)propyle et le (méth)acrylate de 4-(N,N-dipropylamino)butyle ;
- les mono- ou di-(alkyl en C₁-C₄)amino(alkyl en C₁-C₄)-(méth)acrylamides tels que le N'-(2-N,N-diméthylamino)éthyl (méth)acrylamide et le N'-(3-N,N-diméthylamino)propyl acrylamide ;
- les (méth)acrylamides ou (méth)acrylates à groupement hétérocyclique contenant un atome d'azote, tels que le N-(2-pyridyl)acrylamide, le N-(2-imidazolyl)méthacrylamide, le méthacrylate de 2-(4-morpholinyl)éthyle, le acrylate de 2-(4-morpholinyl)éthyle, le N-(4-morpholinyl)méthacrylamide et le N-(4-morpholinyl)acrylamide ; et
- les hétérocycles azotés à groupement(s) vinyle(s) tels que la 2-vinylpyridine et la 4-vinylpyridine.

Lorsque les monomères sont sous forme de sels, il peut s'agir de sels minéraux, tels que les sels chlorhydrate, sulfate et phosphate ; ou bien de sels d'acides organiques, tels que les sels acétate, maléate et fumarate.

Des monomères vinyliques substitués par un ou plusieurs groupes amino particulièrement préférés sont :
- le (méth)acrylate de 3-(N,N-diméthylamino)propyle,
- le N'-(3-N,N-diméthylamino)propyl(méth)acrylamide,
- le (méth)acrylate de 2-(N,N-diméthylamino)éthyle,
- le (méth)acrylate de 2-(N,N-diéthylamino)éthyle,
- le (méth)acrylate de 2-(tert-butylamino)éthyle,
- le 2-(N,N-diméthylamino)propyl(méth)acrylamide, et
- l'acrylate de 2-(N,N-diméthylamino)néopentyle.
   Le ou les monomères vinyliques substitués par un ou plusieurs groupes amino représentent généralement de 10 à 70 % en poids, de préférence de 20 à 60 % en poids, mieux de 30 à 40 % en poids, par rapport au poids total du mélange de monomères.
   Par monomère hydrophobe, on entend au sens de la présente invention, un monomère qui présente une solubilité dans l'eau inférieure à 10g pour 100 mL d'eau à une température de 20°C.
   Le ou les monomères vinyliques non ioniques hydrophobes utilisables pour la préparation du polymère cationique (i) utilisé dans la composition selon l'invention sont généralement choisis parmi les composés répondant à la formule (I) ou (II) :

   (1) CH₂=C(X)Z,

   (II) CH₂=CH-OC(O)R;
dans lesquelles formules (I) et (II) :
X représente H ou un groupe méthyle ;
Z est choisi parmi les groupes -C(O)OR¹, -C(O)NH₂, -C(O)NHR¹, -C(O)N(R¹)₂, -C6H5, -C₆H₄R¹, -C₆H₄OR¹, -C₆H₄Cl, -CN, -NHC(O)CH₃, - NHC(O)H, N-(2-pyrrolidonyle), N-caprolactamyle, -C(O)NHC(CH₃)₃, - C(O)NHCH₂CH₂-NH-CH₂CH₂-urée, -SiR₃, -C(O)O(CH₂)ₓSiR₃, - C(O)NH(CH₂)ₓSiR₃ et -(CH₂)ₓSiR₃ ;
x représente un nombre entier allant de 1 à 6 ;
chaque R représente indépendamment un groupe alkyle en C₁-C₃₀ ;
chaque R¹ représente indépendamment un groupe alkyle en C₁-C₃₀ , un groupe alkyle en C₂-C₃₀ hydroxylé, ou un groupe alkyle en C₁-C₃₀ halogéné.

On peut citer en particulier les (méth)acrylates d'alkyle en C₁-C₃₀ ; les (alkyl en C₁-C₃₀)(méth)acrylamides ; le styrène, les styrènes substitués et en particulier le vinyltoluène (ou 2-méthylstyrène), le butylstyrène, l'isopropylstyrène, le para-chlorostyrène ; les esters de vinyle et en particulier l'acétate de vinyle, le butyrate de vinyle, le caprolate de vinyle, le pivalate de vinyle et le néodécanoate de vinyle ; les nitriles insaturés et en particulier le (méth)acrylonitrile et l'acrylonitrile ; et les silanes insaturés et en particulier le triméthylvinylsilane, le diméthyléthylvinylsilane, l'allyldiméthylphénylsilane, l'allyltriméthylsilane, le 3-acrylamidopropyltriméthylsilane, le méthacrylate de 3-triméthylsilylpropyle.

De préférence, le ou les monomères vinyliques non ioniques hydrophobes sont choisis parmi les esters d'acide acrylique et d'alkyle en C₁-C₃₀, les esters d'acide méthacrylique et d'alkyle en C₁-C₃₀, et leurs mélanges, tels que l'acrylate d'éthyle, le méthacrylate de méthyle, le méthacrylate de 3,3,5-triméthylcyclohexyle, et leurs mélanges.

Le ou les monomères vinyliques non ioniques hydrophobes représentent généralement de 20 à 80% en poids, de préférence de 20 à 70% en poids, mieux de 50 à 65 % en poids, par rapport au poids total du mélange de monomères.

Le ou les monomères vinyliques associatifs utilisables pour la préparation du polymère cationique (i) utilisé dans la composition selon l'invention sont généralement choisis parmi des composés ayant une extrémité (i') à insaturation(s) éthylénique(s) pour la polymérisation par addition avec d'autres monomères du système, une portion centrale (ii') polyoxyalkylène pour conférer des propriétés hydrophiles sélectives aux polymères, et une extrémité (iii') hydrophobe pour conférer des propriétés hydrophobes sélectives aux polymères.

L'extrémité (i') à insaturation(s) éthylénique(s) du ou des monomères vinyliques associatifs est de préférence dérivée d'un acide ou d'un anhydride mono ou di-carboxylique à insaturation(s) α, β-éthylénique(s), de préférence un acide ou un anhydride mono ou di-carboxylique en C₃ ou C₄. De façon alternative, l'extrémité (i') du monomère associatif peut être dérivée d'un éther allylique ou d'un éther vinylique ; d'un monomère non ionique uréthane substitué par un groupe vinyle, tel que divulgué dans le brevet US redélivré n°33,156 ou dans le brevet US 5,294,692 ; ou d'un produit de réaction urée substituée par un groupe vinyle, tel que divulgué dans le brevet US 5,011,978.

La portion centrale (ii') du ou des monomères vinyliques associatifs est de préférence un segment polyoxyalkylène comprenant 5 à 250, de préférence encore 10 à 120, et mieux 15 à 60 motifs oxydes d'alkylène en C₂-C₇. Des portions centrales (ii') préférées sont les segments polyoxyéthylène, polyoxypropylène, et polyoxybutylène comprenant 5 à 150, de préférence 10 à 100, et mieux 15 à 60 motifs oxydes d'éthylène, de propylène ou de butylène, et des séquences aléatoires ou non aléatoires de motifs oxydes d'éthylène, oxydes de propylène ou oxydes de butylène. De préférence, les portions centrales sont des segments polyoxyéthylène.

L'extrémité (iii') hydrophobe du ou des monomères vinyliques associatifs est de préférence un fragment hydrocarboné choisi parmi un groupe alkyle linéaire en C₈-C₄₀, un groupe alkyle en C₂-C₄₀ substitué par un groupe aryle, un groupe phényle substitué par un groupe alkyle en C₂-C₄₀, un groupe alkyle ramifié en C₈-C₄₀, un groupe alicyclique en C₈-C₄₀, et un ester complexe en C₈-C₈₀.

Par ester complexe, on entend au sens de la présente invention, tout ester différent d'un ester simple.

Par ester simple, on entend au sens de la présente invention, tout ester d'alcool aliphatique saturé en C₁-C₃₀ linéaire ou ramifié et non substitué.

Des exemples d'extrémités (iii') hydrophobes du ou des monomères vinyliques associatifs sont des groupes alkyles linéaires ou ramifiés ayant de 8 à 40 atomes de carbone, tels que les groupes capryle (C₈), isooctyle (C₈ ramifié), décyle (C₁₀), lauryle (C₁₂), myristyle (C₁₄), cétyle (C₁₆), cétéaryle (C₁₆-C₁₈), stéaryle (C₁₈), isostéaryle (C₁₈ ramifié), arachidyle (C₂₀), béhényle (C₂₂), lignocéryle (C₂₄), cérotyle (C₂₆), montanyle (C₂₈), mélyssile (C₃₀) et laccéryle (C₃₂).

Des exemples de groupes alkyles linéaires ou ramifiés, ayant 8 à 40 atomes de carbone et dérivés d'une source naturelle sont notamment les groupes alkyles dérivés de l'huile d'arachide hydrogénée, d'huile de soja, d'huile de canola (à prédominance C₁₈), et de l'huile de suif hydrogénée en C₁₆-C₁₈ ; et les terpénols hydrogénés en C₁₀-C₃₀, tels que le géraniol hydrogéné (en C₁₀ ramifié), le farnesol hydrogéné (C₁₅ ramifié) et le phytol hydrogéné (C₂₀ ramifié).

Des exemples de groupe phényle substitué par un groupe alkyle en C₂-C₄₀ sont les groupes octylphényle, nonylphényle, décylphényle, dodécylphényle, hexadécylphényle, octadécylphényle, isooctylphényle et sec-butylphényle.

Des groupes alicycliques en C₈-C₄₀ peuvent être, par exemple, des groupes dérivés de stérols d'origine animale, tels que le cholestérol, le lanostérol, le 7-déhydrocholestérol ; ou bien des groupes dérivés de stérols d'origine végétale, tels que le phytostérol, le stigmastérol et le campestérol ; ou bien les dérivés de stérols issus de microorganismes, tels que l'ergostérol et le mycrostérol. D'autres groupes alicycliques utilisables dans la présente invention sont par exemple les groupes cyclooctyle, cyclododécyle, adamantyle et décahydronaphtyle, et les groupes dérivés de composés alicycliques naturels tels que le pinène, le rétinol hydrogéné, le camphre et l'alcool isobornylique.

Les groupes alkyles en C₂-C₄₀ substitués par un groupe aryle peuvent être, par exemple, un groupe 2-phényléthyle, un groupe 2,4-diphénybutyle, un groupe 2,4,6-triphénylhexyle, un groupe 4-phénylbutyle, un groupe 2-méthyl-2-phényléthyle et un groupe 2,4,6-tri(1'-phényléthyl)phényle.

A titre d'esters complexes en C₈-C₈₀, de préférence en C₈-C₄₀, utilisables comme extrémité (iii'), on peut notamment citer l'huile de ricin hydrogénée (principalement le triglycéride de l'acide 12-hydroxystéarique) ; les 1,2-diacylglycérols tels que le 1,2-distéarylglycérol, le 1,2-dipalmitylglycérol, le 1,2-dimyristylglycérol ; les di-, tri- ou polyesters de sucres tel que le 3,4,6-tristéarylglucose, le 2,3-dilaurylfructose ; et les esters de sorbitane tels que ceux divulgués dans le brevet US 4,600,761.

Les monomères vinyliques associatifs utilisables selon l'invention peuvent être préparés par toute méthode connue dans l'art antérieur. On peut se référer par exemple aux brevets US 4,421,902, US 4,384,096, US 4,514,552, US 4,600,761, US 4,616,074, US 5,294,692, US 5,292,843 ; US 5,770,760 et US 5,412,142.

De préférence, le ou les monomères vinyliques associatifs utilisables selon l'invention sont choisis parmi les composés de formule (III) : 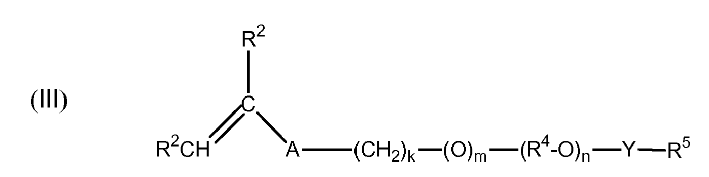 dans laquelle
chaque R² représente indépendamment H, un groupe méthyle, un groupe -C(O)OH, ou un groupe -C(O)OR³ ;
R³ représente un groupe alkyle en C₁-C₃₀ ;
A représente un groupe -CH₂C(O)O-, -C(O)O-, -O-, -CH₂O-, -NHC(O)NH-, -C(O)NH-, -Ar-(CE₂)_{z}-NHC(O)O-, -Ar-(CE₂)_{z}-NHC(O)NH- ou -CH₂CH₂-NHC(O)- ;
Ar représente un groupe arylène ;
E représente H ou un groupe méthyle ;
z est égal à 0 ou 1 ;
k est un nombre entier allant de 0 à 30 ;
m est égal à 0 ou 1, à la condition que lorsque k = 0, m = 0, et lorsque k varie de 1 à 30, m est égal à 1 ;
(R⁴-O)ₙ représente un groupement polyoxyalkylène, qui est un homopolymère, un copolymère aléatoire, ou un copolymère à blocs, comportant des motifs oxyalkylène en C₂-C₄,
R⁴ représente -C₂H₄-, -C₃H₆-, -C₄H₈- ou leurs mélanges,
n est un entier variant de 5 à 250,
Y rerpésente -R⁴O-, -R⁴NH-, -C(O)-, -C(O)NH-, R⁴NHC(O)NH-, ou -C(O)NHC(O)- ;

R⁵ représente un groupe alkyle substitué ou non, choisi parmi les groupes alkyles linéaires en C₈-C₄₀, les groupes alkyles ramifiés en C₈-C₄₀, les groupes alicycliques en C₈-C₄₀, les groupes phényles substitués par un groupe alkyle en C₂-C₄₀, les groupes alkyles en C₂-C₄₀ substitués par un groupe aryle, et les esters complexes en C₈-C₈₀,
le groupe alkyle R⁵ comprenant éventuellement un ou plusieurs substituants choisis parmi les groupes hydroxyle, alcoxyle et halogéno.

De préférence, le ou les monomères vinyliques associatifs sont choisis parmi les (méth)acrylates polyéthoxylés de cétyle, les (méth)acrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6-tri(1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges, où la portion polyéthoxylée du monomère comprend de 5 à 100, de préférence de 10 à 80, et mieux de 15 à 60 motifs oxydes d'éthylène.

Plus particulièrement, le ou les monomères vinyliques associatifs sont choisis parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, où la portion polyéthoxylée du monomère comprend de 10 à 80, de préférence de 15 à 60 et mieux de 20 à 40 motifs oxydes d'éthylène.

De préférence le ou les monomères vinyliques associatifs représentent de 0,001 à 25 % en poids, de préférence de 0,01 à 15 % en poids et mieux de 0,1 à 10 % en poids du mélange de monomères.

De préférence, le mélange de monomères pour l'obtention de polymères cationiques (i) contient en outre un ou plusieurs monomères semi-hydrophobes.

Le ou les monomères tensioactifs vinyliques semi-hydrophobes peuvent modérer les propriétés associatives des polymères associatifs cationiques qui les contiennent, produisant ainsi des gels aqueux ayant une très bonne texture et de très bonnes propriétés rhéologiques.

Par monomère tensioactif vinylique semi-hydrophobe, on entend au sens de la présente invention, on entend une structure similaire à un monomère associatif, mais qui a une extrémité substantiellement non hydrophobe et ainsi ne confère pas de propriété associative aux polymères.

La propriété d'associativité d'un polymère est liée à la propriété dans un milieu donné des molécules dudit polymère de s'associer entre elles ou de s'associer à des molécules d'un coagent (en général un tensioactif) ce qui se traduit dans un certain domaine de concentrations à un accroissement supplémentaire de la viscosité du milieu.

Le ou les monomères tensioactifs vinyliques semi-hydrophobes sont généralement des composés ayant deux parties :
(i^{''}) un groupe terminal insaturé pour permettre la polymérisation par addition avec les autres monomères du mélange de réaction, et
(ii^{''}) un groupe polyoxyalkylène pour atténuer les associations entre les groupes hydrophobes du polymère ou les groupes hydrophobes des autres matériaux éventuellement présents dans la composition contenant le polymère.

L'extrémité fournissant l'insaturation vinylique ou éthylénique pour la polymérisation par addition est de préférence dérivée d'un acide ou d'un anhydride mono ou di-carboxylique à insaturation α, β-éthylénique, de préférence un acide mono ou di-carboxylique en C₃-C₄, ou un anhydride de cet acide. De façon alternative, l'extrémité (i^{''}) peut dériver d'un éther allylique, d'un éther vinylique ou d'un uréthane insaturé non ionique.

L'extrémité (i^{''}) insaturée polymérisable peut aussi dériver d'un acide gras insaturé en C₈-C₃₀ contenant au moins un groupe fonctionnel carboxy libre. Ce groupe en C₈-C₃₀ fait partie de l'extrémité insaturée (i^{''}) et est différent des groupes hydrophobes pendant des monomères associatifs, qui sont séparés de l'extrémité insaturée du monomère associatif par un groupe espaceur hydrophile.

La portion (ii^{''}) polyoxyalkylène comprend un segment polyoxyalkylène à chaîne longue, qui est essentiellement similaire à la portion hydrophile des monomères associatifs. Des portions polyoxyalkylène (ii^{''}) préférées incluent les motifs en C₂-C₄ polyoxyéthylène, polyoxypropylène, et polyoxybutylène comprenant de 5 à 250, de préférence de 10 à 100 motifs oxyalkylène. Lorsque le monomère tensioactif vinylique semi-hydrophobe comprend plus d'un type de motif oxyalkylène, ces motifs peuvent être disposés en séquence aléatoire, non aléatoire, ou à bloc.

De préférence, le ou les monomères tensioactifs vinyliques semi-hydrophobes sont choisis parmi les composés de formules (IV) ou (V) : 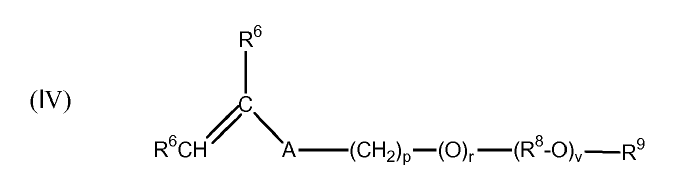 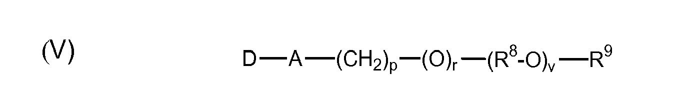 dans lesquelles formules (IV) et (V) :
chaque R⁶ représente indépendamment H, un groupe alkyle en C₁-C₃₀, -C(O)OH, ou -C(O)OR⁷ ;
R⁷ représente un groupe alkyle en C₁-C₃₀ ;
A représente un groupe -CH₂C(O)O-, -C(O)O-, -O-, -CH₂O-, -NHC(O)NH-, -C(O)NH-, -Ar-(CE₂)_{z}-NHC(O)O-, -Ar-(CE₂)_{z}-NHC(O)NH- ou -CH₂CH₂NHC(O)- ;
Ar représente un groupe arylène ;
E représente H ou un groupe méthyle ;
z est égal à 0 ou 1 ;
p est un nombre entier allant de 0 à 30 ;
r est égal à 0 ou 1, à la condition que lorsque p est égal à 0, r est égal à 0, et lorsque p varie de 1 à 30, r est égal 1,
(R₈-O)ᵥ représente un groupement polyoxyalkylène qui est un homopolymère, un copolymère aléatoire, ou un copolymère à blocs avec des motifs oxyalkylène en C₂-C₄, où R⁸ représente -C₂H₄-, -C₃H₆-, - C₄H₈- ou leurs mélanges, et v est un nombre entier allant de 5 à 250 ;
R⁹ représente H ou un groupe alkyle en C₁-C₄ ;

D représente un groupe alcényle en C₈-C₃₀ ou un groupe alcényle en C₈-C₃₀ substitué par un groupe carboxy.

De manière particulièrement préférée, le mélange de monomères comprend un monomère tensioactif vinylique semi-hydrophobe ayant l'une des formules suivantes :

CH₂=CH-O(CH₂)ₐO(C₃H₆O)_{b}(C₂H₄O)_{c}H

ou

CH₂=CHCH₂O(C₃H₆O)_{d}(C₂H₄O)ₑH;

dans lesquelles:
a est égal à 2, 3, ou 4 ;
b est un nombre entier allant de 1 à 10 ;
c est un nombre entier allant de 5 à 50 ;
d est un nombre entier allant de 1 à 10 ; et
e est un nombre entier allant de 5 à 50.

Des monomères tensioactifs vinyliques semi-hydrophobes préférés sont, par exemple, les émulsifiants polymérisables commercialisés sous les références EMULSOGEN^{®} R109, R208, R307, RAL109, RAL208 et RAL307 par la société CLARIANT ; BX-AA-E5P5 commercialisé par la société BIMAX ; et le MAXEMUL^{®} 5010 et 5011 commercialisé par la société UNIQEMA. Les monomères particulièrement préférés sont l'EMULSOGEN^{®} R208, R307 et RAL 307.

Selon les fabricants :
l'EMULSOGEN^{®} R109 est un éther vinylique 1,4-butanediol éthoxylé/propoxylé aléatoire ayant la formule empirique :

   CH₂=CH-O(CH₂)₄O(C₃H₆O)₄(C₂H₄O)₁₀H;
l'EMULSOGEN^{®} R208 qui est un éther vinylique 1,4-butanediol éthoxylé/propoxylé aléatoire ayant la formule empirique :

   CH₂=CH-O(CH₂)₄O(C₃H₆O)₄(C₂H₄O)₂₀H;
l'EMULSOGEN^{®} R307 qui est un éther vinylique 1,4-butanediol éthoxylé/propoxylé aléatoire ayant la formule empirique :

   CH₂=CH-O(CH₂)₄O(C₃H₆O)₄(C₂H₄O)₃₀H;
l'EMULSOGEN^{®} RAL 109 qui est un éther allylique éthoxylé/propoxylé aléatoire ayant la formule empirique :

   CH₂=CHCH₂-O(C₃H₆O)₄(C₂H₄O)₁₀H;
l'EMULSOGEN^{®} RAL 208 qui est un éther allylique éthoxylé/propoxylé aléatoire ayant la formule empirique :

   CH₂=CHCH₂-O(C₃H₆O)₄(C₂H₄O)₂₀H;
l'EMULSOGEN^{®} RAL 307 qui est un éther allylique éthoxylé/propoxylé aléatoire ayant la formule empirique :

   CH₂=CHCH₂-O(C₃H₆O)₄(C₂H₄O)₃₀H;
le MAXEMUL^{®} 5010 qui est un alcényle en C₁₂-C₁₅ carboxylé hydrophobe, éthoxylé avec 24 unités d'oxyde éthylène,
le MAXEMUL^{®} 5011 qui est un alcényle en C₁₂-C₁₅ carboxylé hydrophobe, éthoxylé avec 34 unités d'oxyde éthylène ;
et le BX-AA-E5P5 qui est un éther allylique éthoxylé/propoxylé aléatoire ayant la formule empirique :

   CH₂=CHCH₂-O(C₃H₆O)₅(C₂H₄O)₅H.

La quantité du ou des monomères tensioactifs vinyliques semi-hydrophobes utilisés dans la préparation des polymères cationiques, de préférence épaississants, peut varier largement et dépend, entre autres, des propriétés rhéologiques finales désirées pour le polymère. Elle varie de 0 à 25 % en poids, mieux de 0,01 à 25% en poids, de préférence de 0,1 à 10 % en poids par rapport au poids total du mélange de monomères.

Le ou les polymères cationiques (i) utilisés dans la composition selon l'invention sont préparés à partir d'un mélange de monomères pouvant contenir un ou plusieurs monomères vinyliques non ioniques hydroxylés.

Ces monomères sont des monomères à insaturation éthylénique comprenant un ou plusieurs substituants hydroxyle.

A titre de monomères vinyliques non ioniques hydroxylés, on peut citer les (méth)acrylates d'hydroxyalkyle en C₁-C₆, de préférence les (méth)acrylates d'hydroxyalkyle en C₁-C₄, tel que le méthacrylate de 2-hydroxyéthyle (HEMA), l'acrylate de 2-hydroxyéthyle (2-HEA) et l'acrylate de 3-hydroxypropyle ; les (hydroxyalkyl en C₁-C₄)(méth)acrylamides, tels que le N-(2-hydroxyéthyl)méthacrylamide, le N-(2-hydroxyéthyl)acrylamide, le N-(3-hydroxypropyl)acrylamide et le N-(2,3-dihydroxypropyl)acrylamide ; et leurs mélanges. On peut encore citer l'alcool allylique, l'éther monoallylique de glycérol, le 3-méthyl-3-butèn-1-ol, les précurseurs de l'alcool vinylique et leurs équivalents, tel que l'acétate de vinyle.

Lorsqu'il(s) est (sont) présent(s), le ou les monomères vinyliques non ioniques hydroxylés représentent généralement jusqu'à 10 % en poids du poids total du mélange de monomères. Il(s) représente(nt) donc de 0 à 10 % en poids du poids total du mélange de monomères. De préférence, le ou les monomères vinyliques non ioniques hydroxylés représentent de 0,01 à 10% en poids, mieux de 1 à 8%, et encore de 1 à 5% en poids par rapport au poids total du mélange de monomères.

Le ou les polymères cationiques (i) utilisés dans la composition selon l'invention sont préparés à partir d'un mélange de monomères qui peut comprendre un ou plusieurs monomères réticulants permettant d'introduire des ramifications et de contrôler la masse moléculaire.

Des agents réticulants polyinsaturés utilisables sont bien connus dans l'état de la technique. Des composés mono-insaturés présentant un groupe réactif capable de réticuler un copolymère formé avant, pendant ou après la polymérisation peuvent aussi être utilisés. D'autres monomères réticulants utilisables peuvent être des monomères polyfonctionnels contenant des groupes réactifs multiples tels que des groupes époxydes, isocyanates et des groupes silanes hydrolysables. De nombreux composés polyinsaturés peuvent être utilisés pour générer un réseau tridimensionnel partiellement ou substantiellement réticulé.

Des exemples de monomères réticulants polyinsaturés utilisables sont, par exemple, les monomères aromatiques polyinsaturés, tel que le divinylbenzène, le divinylnaphtylène et le trivinylbenzène ; les monomères alicycliques polyinsaturés, tel que le 1,2,4-trivinylcyclohexane ; les esters difonctionnels de l'acide phtalique tel que le phthalate de diallyle ; les monomères aliphatiques polyinsaturés, tels que les diènes, les triènes et les tétraènes, notamment l'isoprène, le butadiène, le 1,5-hexadiène, le 1,5,9-décatriène, le 1,9-décadiène et le 1,5 heptadiène.

D'autres monomères réticulants polyinsaturés utilisables sont, par exemple, les éthers polyalcényliques, tels que le triallylpentaérythritol, le diallylpentaérythritol, le diallylsaccharose, l'octaallylsaccharose et l'éther diallylique de triméthylolpropane ; les esters polyinsaturés de polyalcools ou de polyacides, tels que le di(méth)acrylate de 1,6-hexanediol, le tri(méth)acrylate de tétraméthylène, l'acrylate d'allyle, l'itaconate de diallyle, le fumarate de diallyle, le maléate de diallyle, le tri(méth)acrylate de triméthylolpropane, le di(méth)acrylate de triméthylolpropane et le di(méth)acrylate de polyéthylène glycol ; les alkylène-bisacrylamides tels que le méthylène-bisacrylamide et le propylène-bisacrylamide ; les dérivés hydroxylés et carboxylés du méthylène-bisacrylamide, tels que le N,N'-bisméthylol-méthylène-bisacrylamide ; les di(méth)acrylates de polyéthylèneglycol, tels que le di(méth)acrylate d'éthylèneglycol, le di(méth)acrylate de diéthylèneglycol et le di(méth)acrylate de triéthylèneglycol ; les silanes polyinsaturés tels que le diméthyldivinylsilane, le méthyltrivinylsilane, l'allyldiméthylvinylsilane, le diallyldiméthylsilane et le tétravinylsilane ; les stannanes polyinsaturés tels que le tétraallylétain et le diallyldiméthylétain.

Des monomères réticulants monoinsaturés utilisables et portant un groupe réactif peuvent être les N-méthylolacrylamides ; les N-alcoxy(méth)acrylamides, où le groupe alcoxy comporte de 1 à 18 atomes de carbone ; et les silanes hydrolysables insaturés tels que triéthoxyvinylsilane, le tris-isopropoxyvinylsilane et le méthacrylate de 3-triéthoxysilylpropyle.

Des monomères réticulants polyfonctionnels utilisables et contenant plusieurs groupes réactifs peuvent être, par exemple, les silanes hydrolysables tels que l'éthyltriéthoxysilane et l'éthyltriméthoxysilane ; les silanes hydrolysables époxydés tels que le 2-(3,4-époxycyclohexyl)éthyltriéthoxysilane et le 3-glycidoxypropyl-triméthyoxysilane ; les polyisocyanates tels que le 1,4-diisocyanato-butane, le 1,6-diisocyanatohexane, le 1,4-phénylènediisocyanate et le 4,4'-oxybis(phénylisocyanate) ; les époxydes insaturés tels que le méthacrylate de glycidyle et l'allylglycidyléther ; les polyépoxydes tels que le diglycidyléther, le 1,2,5,6-diépoxyhexane, et l'éthylèneglycoldiglycidyléther.

Des monomères réticulants polyinsaturés particulièrement utilisables sont les polyols éthoxylés, tels les diols, les triols et les bis-phénols, éthoxylés avec 2 à 100 moles d'oxyde d'éthylène par mole de groupe fonctionnel hydroxyle et terminés par un groupe insaturé polymérisable tel qu'un vinyléther, un allyléther, un ester acrylate ou un ester méthacrylate. De tels monomères réticulants peuvent être par exemple le diméthacrylate éthoxylé de biphénol A, le diméthacrylate éthoxylé de biphénol F, et le triméthacrylate éthoxylé de triméthylol propane.

D'autres monomères réticulants éthoxylés utilisables dans la présente invention sont, par exemple, les agents réticulants dérivés des polyols éthoxylés divulgués dans le brevet US 6 140 435.

Des exemples particulièrement préférés de monomères réticulants sont des esters acrylates et méthacrylates de polyols ayant au moins deux groupes ester acrylate ou méthacrylate, tel que le triacrylate de triméthylolpropane (TMPTA), le diméthacrylate de triméthylolpropane, le diméthacrylate de triéthylène glycol (TEGDMA), et le diméthacrylate de bisphénol A éthoxylé (30) (EOBDMA).

Lorsqu'il(s) est (sont) présent(s), le ou les monomères réticulants représentent de préférence au plus 5% en poids par rapport au poids du mélange de monomères. Selon un mode de réalisation préféré, les monomères réticulants sont présents à une teneur allant de 0,001 à 5% en poids, de préférence de 0,05 à 2% en poids, mieux de 0,1 à 1% en poids par rapport au poids total du mélange de monomères.

Le mélange de monomères peut contenir en outre un ou plusieurs agents de transfert de chaîne. Les agents de transferts de chaîne sont des composés bien connus de l'état de la technique.

On peut citer en particulier les composés thiolés, les composés disulfures, tels que les mercaptans en C₁-C₁₈, les acides mercaptocarboxyliques, les esters d'acides mercaptocarboxyliques, les thioesters, les (alkyl en C₁-C₁₈)disulfures, les aryldisulfures, les thiols polyfonctionnels ; les phosphites et hypophosphites ; les composés halogénoalcanes, tel que le tétrachlorure de carbone, le bromotrichlorométhane ; et les agents de transfert de chaîne insaturés, tel que l'alpha-méthylstyrène.

Les thiols polyfonctionnels sont, par exemple, les thiols trifonctionnels, tel que le triméthylolpropane-tris-(3-mercaptopropionate), les thiols tétrafonctionnels, tel que le pentaérythritol-tétra-(3-mercaptopropionate), le pentaérythritol-tétra-(thioglycolate) et le pentaérythritol-tétra(thiolactate) ; les thiols hexafonctionnels, tel que le pentaérythritol-hexa-(thioglyconate).

De façon alternative, le ou les agents de transfert de chaîne peuvent être des agents de transfert de chaîne catalytiques qui réduisent le poids moléculaire des polymères d'addition lors de la polymérisation par radicaux libres des monomères vinyliques. On peut citer par exemple les complexes de cobalt, notamment les chélates de cobalt (II). Les agents de transfert de chaîne catalytiques peuvent souvent être utilisés à des concentrations faibles par rapport aux agents de transfert de chaîne thiolés.

De façon particulièrement préférée, on peut citer à titre d'agent de transfert de chaîne l'octyl mercaptan, le n-dodécyle mercaptan, le t-dodécyle mercaptan, l'hexadécyle mercaptan, l'octadécyle mercaptan (ODM), l'isooctyl 3-mercaptopropionate (IMP), le butyl 3-mercaptopropionate, l'acide 3-mercaptopropionique, le butyl thioglycolate, l'isooctyl thioglycolate, le dodécyle thioglycolate.

Lorsqu'il(s) est (sont) présents, le ou les agents de transferts de chaîne sont ajoutés au mélange de monomères de préférence jusqu'à 10% en poids par rapport au poids total du mélange de monomères. De préférence, le ou les agents de transfert de chaîne représentent de 0,1% à 5 % en poids par rapport au poids total de monomères.

Le mélange de monomères permettant la préparation du polymère cationique (i) utilisé dans la composition selon l'invention peut comprendre un ou plusieurs agents de stabilisation polymériques pour l'obtention de dispersions ou d'émulsions stables. De préférence, les polymères stabilisants sont solubles dans l'eau. On peut citer par exemple les polymères synthétiques, tels que les alcools polyvinyliques, les acétates polyvinyliques partiellement hydrolysés, la polyvinylpyrrolidone, les polyacrylamides, les polyméthacrylamides, les polymères d'addition carboxylés, les polyalkyles vinyle éthers ; les polymères naturels hydrosolubles, tels que la gélatine, les peptines, les alginates, la caséine ; les polymères naturels modifiés, tels que la méthylcellulose, l'hydroxypropylcellulose, la carboxyméthylcellulose, les hydroxyéthylcelluloses allyliques.

Les agents de stabilisation polymériques sont utilisés en une quantité au plus égale à 2 % en poids par rapport au poids total du mélange de monomères, de préférence en une quantité comprise 0,0001 et 1 % en poids, mieux entre 0,01 et 0,5 % en poids par rapport au poids total du mélange de monomères.

Selon un mode de réalisation préféré, le mélange de monomères comprend, par rapport au poids total du mélange de monomères :
a) de 10 à 70 % en poids d'un ou plusieurs monomères vinyliques substitués par un ou plusieurs groupes amino,
b) de 20 à 80 % en poids d'un ou plusieurs monomères vinyliques non ioniques hydrophobes,
c) de 0,001 à 25 % en poids d'un ou plusieurs monomères vinyliques associatifs,
d) de 0 à 25 % en poids d'un ou plusieurs monomères tensioactifs vinyliques semi-hydrophobes,
e) de 0 à 10 % en poids d'un ou plusieurs monomères vinyliques non ioniques hydroxylés,
f) de 0 à 5% en poids d'un ou plusieurs monomères réticulants,
g) de 0 à 10 % en poids d'un ou plusieurs agents de transfert de chaîne, et
h) de 0 à 2 % en poids d'un ou plusieurs agents de stabilisation polymérique.

De façon encore plus préférée, le mélange de monomères comprend, par rapport au poids total du mélange de monomères :
a) de 20 à 60 % en poids du ou des monomères vinyliques substitués par un ou plusieurs groupes amino,
b) de 20 à 70 % en poids du ou des monomères vinyliques non ioniques hydrophobes,
c) de 0,01 à 15 % en poids du ou des monomères vinyliques associatifs,
d) de 0,1 à 10 % en poids du ou des monomères tensioactifs vinyliques semi-hydrophobes,
e) de 0,01 à 10 % en poids du ou des monomères vinyliques non ioniques hydroxylés,
f) de 0,001 à 5 % en poids du ou des monomères réticulants,
g) de 0,001 à 10 % en poids du ou des agents de transfert de chaîne, et
h) de 0 à 2 % en poids du ou des agents de stabilisation polymériques.

Selon un mode de réalisation particulièrement préféré, le mélange de monomères permettant la préparation du polymère cationique (i) utilisé dans la composition selon l'invention comprend, par rapport au poids total du mélange de monomères :
a) de 20 à 50 % en poids d'un ou plusieurs monomères vinyliques substitués par un ou plusieurs groupes amino choisi parmi :
   - le (méth)acrylate de 3-(N,N-diméthylamino)propyle,
   - le N'-(3-N,N-diméthylamino)propyl(méth)acrylamide,
   - le (méth)acrylate de 2-(N,N-diméthylamino)éthyle,
   - le (méth)acrylate de 2-(N,N-diéthylamino)éthyle,
   - le (méth)acrylate de 2-(tert-butylamino)éthyle,
   - le 2-(N,N-diméthylamino)propyl (méth)acrylamide, et
   - l'acrylate de 2-(N,N-diméthylamino)néopentyle,
b) de 50 à 65 % en poids d'un ou plusieurs monomères vinyliques non ioniques hydrophobes choisis parmi les esters d'acide acrylique et d'alkyle en C₁-C₃₀, les esters de l'acide méthacrylique et d'alkyle en C₁-C₃₀, et leurs mélanges,
c) de 0,1 à 10 % en poids d'un ou plusieurs monomères vinyliques associatifs choisis parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6-tri(1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges,
d) de 0,1 à 10 % en poids d'un ou plusieurs monomères tensioactifs vinyliques semi-hydrophobes ayant l'une des formules suivantes :

   CH₂=CH-O(CH₂)ₐO(C₃H₆O)_{b}(C₂H₄O)_{c}H

   ou

   CH₂=CHCH₂O(C₃H₆O)_{d}(C₂H₄O)ₑH

   dans lesquelles:
   a est égal à 2, 3, ou 4 ;
   b est un nombre entier allant de 1 à 10 ;
   c est un nombre entier allant de 5 à 50 ;
   d est un nombre entier allant de 1 à 10 ; et
   e est un nombre entier allant de 5 à 50,
e) jusqu'à 10 % en poids d'un ou plusieurs monomères vinyliques non ioniques hydroxylés,
f) jusqu'à 5% en poids d'un ou plusieurs monomères réticulants,
g) jusqu'à 10 % en poids d'un ou plusieurs agents de transfert de chaîne, et
h) jusqu'à 2 % en poids d'un ou plusieurs agents de stabilisation polymérique.

Les polymères cationiques (i) encore plus préférés selon l'invention sont des polymères issus de la polymérisation du mélange de monomères suivants :
- un méthacrylate de di(alkyl en C₁-C₄)amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé avec 20 à 30 moles d'oxyde d'éthylène,
- un allyl éther de polyéthylèneglycol/polypropylèneglycol 30/5,
- un méthacrylate d'hydroxy(alkyle en C₂-C₆),
- un diméthacrylate d'éthylèneglycol.

Parmi les polymères cationiques (i) utilisés dans la composition selon l'invention, on peut notamment citer le composé commercialisé par la société NOVEON sous la dénomination AQUA CC et qui correspond à la dénomination INCI POLYACRYLATE-1 CROSSPOLYMER.

Le POLYACRYLATE-1 CROSSPOLYMER est le produit de la polymérisation d'un mélange de monomères comprenant :
- un méthacrylate de di(alkyl en C₁-C₄) amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé (20-25 moles de motif oxyde d'éthylène),
- un allyl éther de polyéthylèneglycol/polypropylèneglycol 30/5,
- un méthacrylate d'hydroxy(alkyle en C₂-C₆), et
- un diméthacrylate d'éthylèneglycol.

Le ou les polymères cationiques (i) utilisés dans les compositions selon l'invention représentent généralement de 0,01 à 10% en poids, de préférence de 0,05 à 5% en poids, et mieux de 0,1 à 1% en poids par rapport au poids total de la composition.

Le ou les polymères cationiques (i) utilisés dans la composition selon l'invention peuvent être préparés par des techniques de polymérisation conventionnelles, telles que la polymérisation en émulsion, comme cela est bien connu dans le domaine des polymères. La polymérisation peut être effectuée par simple procédé discontinu, par procédé par addition contrôlée, ou bien la réaction peut être initiée dans un petit réacteur puis alors la masse des monomères peut être ajoutée de façon contrôlée dans le réacteur (procédé par ensemencement). Généralement, la polymérisation est menée à une température de réaction comprise entre 20 et 80°C, même si des températures supérieures ou inférieures peuvent être utilisées. Pour faciliter l'émulsification du mélange de monomères, la polymérisation par émulsion est effectuée en présence d'un tensioactif présent en une quantité variant de 1 à 10 % en poids, de préférence de 3 à 8% en poids, mieux de 5 à 7 % en poids, par rapport au poids total de l'émulsion. Le milieu de réaction de polymérisation par émulsion comprend également un ou plusieurs initiateurs radicalaires, de préférence en une quantité variante de 0,01 à 3 % en poids par rapport au poids total du mélange de monomères. La polymérisation peut être réalisée dans un milieu aqueux ou bien hydroalcoolique à un pH neutre ou faiblement alcalin.

Dans une polymérisation typique, le mélange de monomères est ajouté sous agitation à une solution de tensioactifs émulsifiants, tel qu'un tensioactif non ionique, de préférence un éthoxylate d'alcool linéaire ou ramifié, ou un mélange de tensioactifs non ioniques et anioniques, tels que des sulfates d'alcool gras ou des alkyl sulfonates d'alcool gras, dans une quantité adaptée d'eau, dans un réacteur adapté, pour préparer l'émulsion de monomères. L'émulsion est désoxygénée au moyen de toute méthode connue, puis la réaction de polymérisation est initiée en ajoutant un catalyseur de polymérisation (amorceur) tel que le persulfate de sodium, ou tout autre catalyseur de polymérisation par addition adaptée, comme cela est bien connu dans le domaine des polymères. Le mélange réactionnel est agité jusqu'à ce que la polymérisation soit complète, généralement pendant une durée variant de 4 heures à 16 heures. L'émulsion de monomères peut être chauffée à une température comprise entre 20 et 80°C avant l'addition de l'amorceur, si cela est souhaité. La quantité de monomères n'ayant pas réagi peut être éliminée par addition d'une quantité supplémentaire de catalyseur. L'émulsion de polymère obtenue peut être retirée du réacteur et emballée pour être stockée ou utilisée. De façon optionnelle, le pH ou d'autres caractéristiques physiques ou chimiques de l'émulsion peuvent être ajustés avant de retirer l'émulsion du réacteur. Généralement, l'émulsion produite a une teneur totale en matières solides qui varie entre 10 et 40 % en poids. Généralement, la quantité totale de polymères dans l'émulsion obtenue varie entre 15 et 35% en poids, en général au plus 25 % en poids.

Des tensioactifs adaptés pour faciliter la polymérisation par émulsion peuvent être des tensioactifs non ioniques, anioniques, amphotères ou cationiques, ou leurs mélanges. Le plus souvent, des tensioactifs non ioniques ou anioniques, ou leurs mélanges sont utilisés.

Tous types de tensioactifs non ioniques, anioniques, amphotères ou cationiques classiquement utilisés dans les polymérisations en l'émulsion peuvent être utilisés.

Comme expliqué plus haut, la polymérisation peut être effectuée en présence d'un ou plusieurs amorceurs conduisant à la formation de radicaux libres. Ceux-ci peuvent être choisis parmi les composés persulfates inorganiques insolubles, tels que le persulfate d'ammonium, le persulfate de potassium, le persulfate de sodium ; les peroxydes tels que le peroxyde d'hydrogène, le peroxyde de benzoyle, le peroxyde d'acétyle, et le peroxyde de lauryle ; les hydroperoxydes organiques, tels que l'hydroperoxyde de cumen et l'hydroperoxyde de t-butyle ; les peracides organiques, tel que l'acide peracétique ; et les agents producteurs de radicaux libres solubles dans l'huile, tels que 2,2'-azobisisobutyronitrile, et leurs mélanges. Les peroxydes et les peracides peuvent être éventuellement activés avec des agents réducteurs, tel que le bisulfite de sodium ou l'acide ascorbique, les métaux de transition, l'hydrazine. Des initiateurs de radicaux libres particulièrement adaptés sont les initiateurs de polymérisation azoïque solubles dans l'eau, tels que les composés 2,2'-azobis(tert-alkyl) ayant un substituant hydrosolubilisant sur le groupe alkyle. Des catalyseurs de polymérisation azoïque préférés sont les initiateurs de radicaux libres VAZO^{®}, commercialisés par la société DuPont, tel que le VAZO^{®}44 (2,2'-azobis(2-4,5-dihydroimidazolyl)propane), le VAZO^{®}56 (2,2'-azobis(2-méthylpropio-namidine)dihydrochlorure), et le VAZO^{®}68 (4,4'-azobis(acide 4-cyanovalérique)).

Les compositions de l'invention comprennent un ou plusieurs tensioactifs choisis parmi les tensioactifs anioniques, cationiques, amphotères et non ioniques.

Les tensioactifs anioniques pouvant être utilisés dans les compositions de l'invention sont notamment choisis parmi les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkylamidoéthersulfates, les alkylarylpolyéthersulfates, les monoglycéride-sulfates, les alkylsulfonates, les alkylamidesulfonates, les alkylarylsulfonates, les α-oléfine-sulfonates, les paraffine-sulfonates, les alkylsulfosuccinates, les alkyléthersulfosuccinates, les alkylamide-sulfosuccinates, les alkylsulfo-acétates, les acylsarcosinates et les acylglutamates, les groupes alkyle et acyle de tous ces composés comportant de 6 à 24 atomes de carbone et le groupe aryle désignant de préférence un groupe phényle ou benzyle.

On peut également utiliser les monoesters d'alkyle en C₆₋₂₄ et d'acides polyglycoside-dicarboxyliques tels que les glucoside-citrates d'alkyle, les polyglycoside-tartrates d'alkyle et les polyglycoside-sulfosuccinates d'alkyle, les alkylsulfosuccinamates, les acyliséthionates et les N-acyltaurates, le groupe alkyle ou acyle de tous ces composés comportant de 12 à 20 atomes de carbone.

Un autre groupe d'agents tensioactifs anioniques utilisables dans les compositions de la présente invention est celui des acyl-lactylates dont le groupe acyle comporte de 8 à 20 atomes de carbone.

En outre, on peut encore citer les acides alkyl-D-galactoside-uroniques et leurs sels ainsi que les acides (alkyl en C₆₋₂₄)éther-carboxyliques polyoxyalkylénés, les acides (alkyl en C₆₋₂₄)(aryl en C₆₋₂₄)éther-carboxyliques polyoxyalkylénés, les acides (alkyl en C₆₋₂₄)amidoéther-carboxyliques polyoxyalkylénés et leurs sels, en particulier ceux comportant de 2 à 50 motifs oxyde d'éthylène, et leurs mélanges.

On utilise de préférence les alkylsulfates, les alkyléthersulfates et leurs mélanges, en particulier sous forme de sels de métaux alcalins ou alcalino-terreux, d'ammonium, d'amine ou d'aminoalcool.

Lorsqu'ils sont présents, la quantité du ou des tensioactifs anioniques est de préférence comprise dans l'intervalle allant de 0,1 à 50 % en poids, mieux encore allant de 1 à 30 % en poids, mieux de 4 à 20% en poids par rapport au poids total de la composition.

Des exemples de tensioactifs non-ioniques additionnels utilisables dans les compositions de la présente invention sont décrits par exemple dans "Handbook of Surfactants" par M.R. PORTER, éditions Blackie & Son (Glasgow and London), 1991, pp 116-178. Ils sont choisis notamment parmi les alcools, les alpha-diols, les alkyl(C₁₋₂₀)phénols ou les acides gras polyéthoxylés, polypropoxylés ou polyglycérolés, ayant une chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras ; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras du sorbitan éthoxylés ayant de 2 à 30 motifs d'oxyde d'éthylène, les esters d'acides gras du saccharose, les esters d'acides gras du polyéthylèneglycol, les (alkyl en C₆₋₂₄)polyglycosides, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

Lorsqu'ils sont présents, la quantité du ou des tensioactifs non ioniques est de préférence comprise dans l'intervalle allant de 0,01 à 25% en poids, mieux encore allant de 0,1 à 20 % en poids par rapport au poids total de la composition.

Les agents tensioactifs amphotères ou zwittérioniques, utilisables dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer en particulier les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3^{ème} édition, 1982, sous les dénominations Amphocarboxy-glycinate et Amphocarboxypropionate de structures respectives (A) et (B) :

Rₐ-CONHCH₂CH₂-N(R_{b})(R_{c})(CH₂COO⁻) (A)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

   Rₐ'-CONHCH2CH2-N(B)(B') (B)

   dans laquelle :
   B représente -CH₂CH₂OX',
   B' représente -(CH₂)_{z}-Y', avec z = 1 ou 2,
   X' représente le groupe -CH₂CH₂-COOH ou un atome d'hydrogène,
   Y' représente -COOH ou le groupe -CH₂-CHOH-SO₃H,
   Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Parmi les tensioactifs amphotères ou zwittérioniques cités ci-dessus, on utilise de préférence les (alkyl en C₈₋₂₀)-bétaïnes, les (alkyl en C₈₋₂₀)-amido(alkyl en C₆₋₈)bétaïnes et leurs mélanges.

Lorsqu'ils sont présents, la quantité du ou des tensioactifs amphotères ou zwittérioniques est de préférence comprise dans l'intervalle allant de 0,01 à 25% en poids, mieux encore de 0,1 à 20% en poids par rapport au poids total de la composition.

Dans une variante préférée, la composition selon l'invention comprend au moins un tensioactif anionique et au moins un tensioactif amphotère ou zwittérionique.

La composition selon l'invention présente de préférence une teneur totale en tensioactifs anioniques, non-ioniques, amphotères et zwittérioniques comprise dans l'intervalle allant de 1 à 50 % en poids, mieux encore allant de 4 à 30 % en poids, par rapport au poids total de la composition.

A titre d'exemples de tensioactif cationique, on peut notamment citer les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium ; les dérivés d'imidazoline ; ou les oxydes d'amines à caractère cationique.

Lorsque les tensioactifs cationiques sont présents, leur quantité est de préférence comprise dans l'intervalle allant de 0,01 à 20 % en poids, mieux encore de 0,1 à 15 % en poids, et encore plus préférentiellement de 0,2 à 10 % en poids par rapport au poids total de la composition cosmétique.

Les compositions selon la présente invention comprennent en outre un ou plusieurs polymères cationiques ou amphotères différents des polymères (i) tels que par exemple ceux décrits dans les brevets français N°2788974 et 2788976 et tels que décrits ci-après.

Ces polymères sont par exemple des polymères hydrosolubles ou hydrodispersibles.

Par polymères hydrosolubles ou hydrodispersibles, on entend au sens de la présente invention, des polymères ayant une solubilité dans l'eau mesurée à 25°C au moins égale à 0,1 gramme/litre (g/L) (obtention d'une solution macroscopiquement isotrope et transparente, colorée ou non). Cette solubilité est de préférence supérieure ou égale à 1 g/L.

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire.

Ce sont des produits connus. Ils sont notamment décrits dans les brevets français n° 2 505 348 ou 2 542 997. Parmi lesdits polymères, on peut citer :
(1) Les homopolymères ou copolymères dérivés d'esters ou d'amides acryliques ou méthacryliques et comportant au moins un des motifs de formules (VI), (VII), (VIII) ou (IX) suivantes: 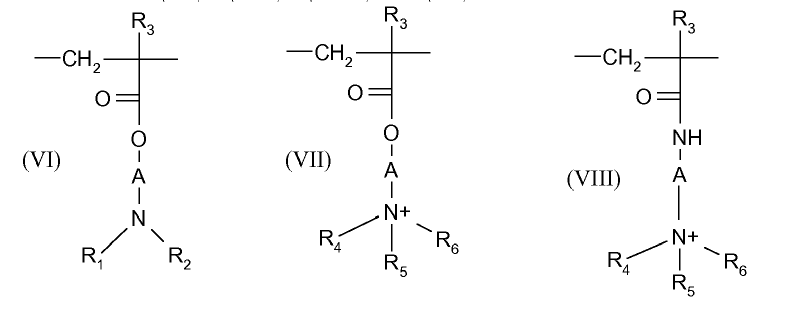 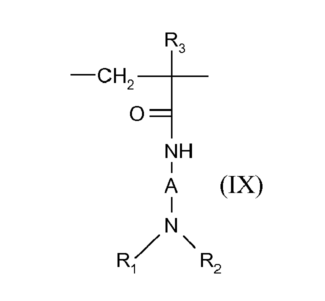 dans lesquelles:
   R₃, identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.
   Les polymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
   Ainsi, parmi ces polymères de la famille (1), on peut citer :
   - les copolymères d'acrylamide et de diméthylaminoéthyl méthacrylate quaternisé au sulfate de diméthyle ou avec un halogénure de diméthyle;
   - les copolymères d'acrylamide et de chlorure de méthacryloyloxyéthyltriméthylammonium décrits par exemple dans la demande de brevet EP-A-080976;
   - le copolymère d'acrylamide et de méthosulfate de méthacryloyloxyéthyltriméthylammonium;
   - les copolymères vinylpyrrolidone / acrylate ou méthacrylate de dialkylaminoalkyle quaternisés ou non. Ces polymères sont décrits en détail dans les brevets français 2.077.143 et 2.393.573;
   - les terpolymères méthacrylate de diméthyl amino éthyle/ vinylcaprolactame/ vinylpyrrolidone;
   - les copolymère vinylpyrrolidone / méthacrylamidopropyl diméthylamine;
   - les copolymères vinylpyrrolidone / méthacrylamide de diméthylaminopropyle quaternisés
   - les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société CIBA.
(2) Les polysaccharides cationiques et en particulier ceux choisis parmi :
   (a) les dérivés d'éthers de cellulose comportant des groupements ammonium quaternaire décrits dans le brevet français 1 492 597. Ces polymères sont également définis dans le dictionnaire CTFA comme des ammonium quaternaires d'hydroxyéthylcellulose ayant réagi avec un époxyde substitué par un groupement triméthylammonium.
   (b) les copolymères de cellulose ou les dérivés de cellulose greffés avec un monomère hydrosoluble d'ammonium quaternaire, et décrits notamment dans le brevet US 4 131 576, tels que les hydroxyalkylcelluloses, comme les hydroxyméthyl-, hydroxyéthyl- ou hydroxypropylcelluloses greffées notamment avec un sel de méthacryloyléthyl triméthylammonium, de méthacrylmidopropyl triméthylammonium, de diméthyl-diallylammonium.
   (c) les polygalactomannanes cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
(3) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(4) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508.
(5) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine.
(6) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
(7) Les cyclopolymères d'alkyl diallyl amine ou de dialkyl diallyl ammonium tels que les homopolymères ou copolymères comportant comme constituant principal de la chaîne des motifs répondant aux formules (X) ou (XI) : 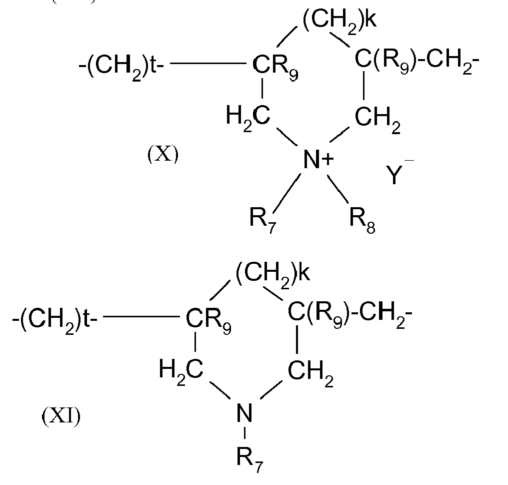 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
(8) Le polymère de diammonium quaternaire contenant des motifs récurrents répondant à la formule : 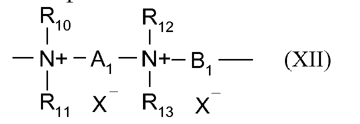 formule (XII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁, R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel n est compris entre 1 et 100 et de préférence entre 1 et 50, et D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- .
   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont constitués de motifs récurrents répondant à la formule (XIII) suivante: 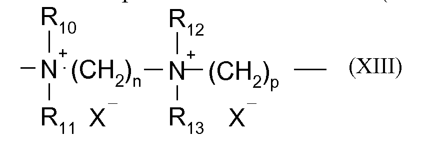 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
(9) Les polymères de poly(ammonium quaternaire) constitués de motifs récurrents de formule (XIV) : 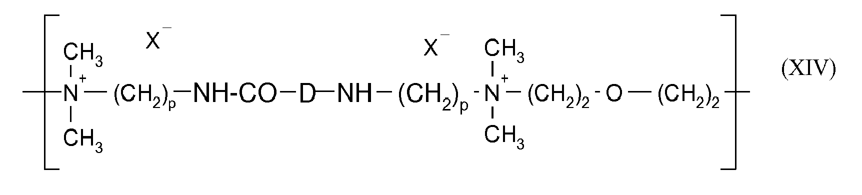 dans laquelle p désigne un nombre entier variant de 1 à 6 environ, D peut être nul ou peut représenter un groupement -(CH₂)ᵣ -CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
(10) Les polymères quaternaires de vinylpyrrolidone et de vinylimidazole.
(11) Les polyamines comme le produit référencé sous le nom de " POLYETHYLENEGLYCOL (15) TALLOW POLYAMINE " dans le dictionnaire CTFA.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Les polymères amphotères utilisables dans les compositions la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;

K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylamino-alkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl triméthyl ammonium.
   Le composé vinylique peut être également un sel de dialkyldiallylammonium tel que le chlorure de diméthyldiallylammonium.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylaminoéthyle avec le sulfate de diméthyle ou diéthyle.
   Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
   Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
   Les comonomères basiques préférés sont des méthacrylates d'aminoéthyle, de butyl aminoéthyle, de N,N'-diméthylaminoéthyle, de N-tertio-butylaminoéthyle.
(3) Les polyaminoamides réticulés et alcoylés partiellement ou totalement dérivant de polyaminoamides de formule générale : 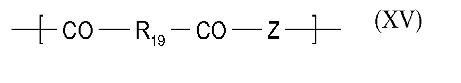 dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 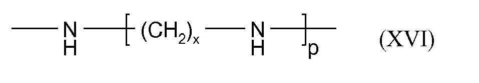 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XVI) ci-dessus, dans lequel x=2 et p=1 et qui dérive de l'éthylènediamine, ou le radical dérivant de la pipérazine : 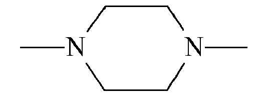
   c) dans les proportions de 0 à 20 moles % le radical -NH-(CH₂)₆-NH- dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
   Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les acides acrylique, méthacrylique, itaconique.
   Les alcanes sultones utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 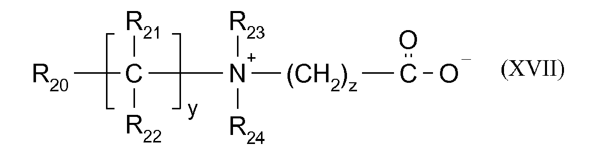 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de butyle / méthacrylate de diméthylcarboxyméthylammonio-éthyle tel que le produit vendu sous la dénomination DIAFORMER Z301 par la société SANDOZ.
(5) les polymères dérivés du chitosane comportant des motifs monomères répondant aux formules (XVIII), (XIX), (XX) suivantes : 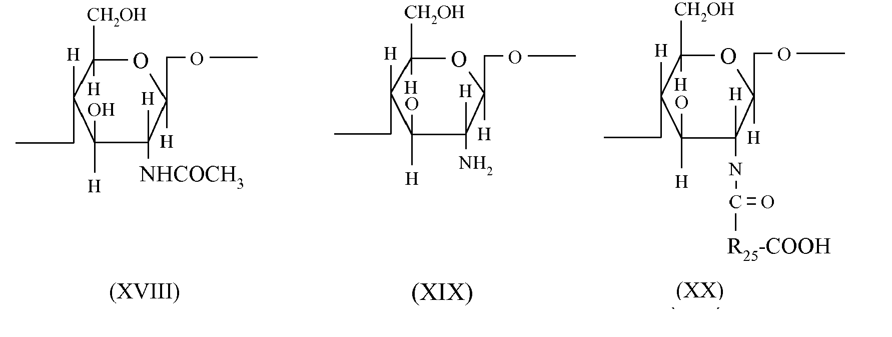 le motif (XVIII) étant présent dans des proportions comprises entre 0 et 30%, le motif (XIX) dans des proportions comprises entre 5 et 50% et le motif (XX) dans des proportions comprises entre 30 et 90%, étant entendu que dans ce motif (XX), R₂₅ représente un radical de formule : 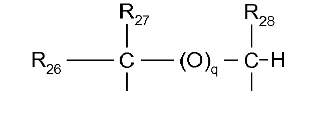 dans laquelle q désigne zéro ou 1 ;
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases
   ou des acides.
(6) Les polymères dérivés de la N-carboxyalkylation du chitosane comme le N-carboxyméthyl chitosane ou le N-carboxybutyl chitosane.
(7) Les polymères répondant à la formule générale (XXI) tels que ceux décrits par exemple dans le brevet français 1 400 366 et comprenant des unités : 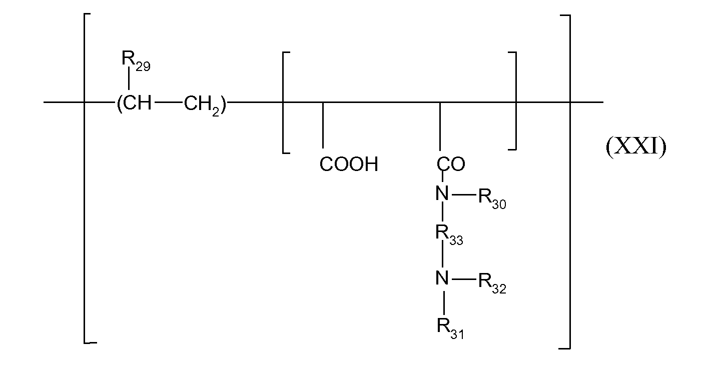 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁ désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂- , -CH₂-CH₂-CH₂- , -CH₂-CH(CH₃)- , R₃₁ ayant les significations mentionnées ci-dessus,
   ainsi que les homologues supérieurs de ces radicaux et contenant jusqu'à 6 atomes de carbone,
   r est tel que le poids moléculaire est compris entre 500 et 6000000 et de préférence entre 1000 et 1000000.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XXII)

      où D désigne un radical 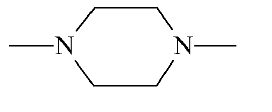 et X désigne le symbole E ou E', E ou E' identiques ou différents désignent un radical bivalent qui est un radical alkylène à chaîne droite
      ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XXIII)
   où D désigne un radical 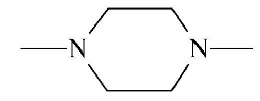 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Parmi tous les polymères cationiques ou amphotères (iii) utilisables selon la présente invention, on préfère notamment :
(a) parmi les polymères cationiques :
   - les dérivés d'éther de cellulose quaternaires tels que les produits commercialisés sous la dénomination « JR 400 » par la société AMERCHOL, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 550 » et « MERQUAT S » par la société MERCK, les polysaccharides cationiques et plus particulièrement les gommes de guar modifiées par du chlorure de 2,3-époxypropyl triméthylammonium commercialisées par exemple sous la dénomination « JAGUAR C13S » par la société AMERCHOL, les homopolymères et les copolymères éventuellement réticulés de sel de (méth)acryloyloxyéthyltriméthylammonium, vendus par la société CIBA en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium), les copolymères quaternaires de vinylpyrrolidone et de sel de vinyl imidazole tels que les produits commercialisés par BASF sous les dénominations LUVIQUAT FC 370, LUVIQUAT FC 550, LUVIQUAT FC 905 et LUVIQUAT HM-552.
(b) parmi les polymères amphotères :
   - le copolymère chlorure de diméthyldiallylammonium/acide acrylique (80/20) commercialisé sous la dénomination MERQUAT 280 DRY par la société NALCO (dénomination CTFA : POLYQUATERNIUM 22);
   - le copolymère chlorure de diméthyldiallylammonium/acide acrylique (95/5) commercialisé sous la dénomination MERQUAT 295 DRY par la société NALCO (dénomination CTFA : POLYQUATERNIUM 22);
   - le copolymère de chlorure de méthacrylamidopropyltrimonium, d'acide acrylique et d'acrylate d'éthyle, commercialisé sous la dénomination MERQUAT 2001 par la société NALCO (dénomination CTFA : POLYQUATERNIUM 47); et
   - le terpolymère acrylamide/chlorure de diméthyldiallylammonium/acide acrylique, commercialisé sous la dénomination MERQUAT PLUS 3330 DRY par la société NALCO (dénomination CTFA : POLYQUATERNIUM 39).

Lorsqu'ils ils sont présents dans les compositions selon la présente invention, les polymères cationiques et/ou amphotères (iii) sont présents dans une proportion pondérale allant de 0,01 à 10% en poids par rapport au poids total de la composition, et de préférence allant de 0,1 à 5 % par rapport au poids total de la composition.

La ou les particules d'un ou plusieurs composés minéraux utilisées dans la composition cosmétique selon l'invention peuvent être choisies parmi les particules métalliques, les oxydes, les sels inorganiques, les carbures, les nitrures, les sulfures et les hydroxydes.

Par particules métalliques, on entend des particules formées par des métaux choisis parmi les métaux alcalino-terreux, les métaux de transition, les métaux des terres rares et les alliages de ces métaux.

De préférence, les métaux utilisés sont en particulier l'aluminium, le cuivre, le cadmium, le sélénium, l'argent, l'or, l'indium, le fer, le platine, le nickel, le molybdène, le silicium, le titane, le tungstène, l'antimoine, le palladium, le zinc, l'étain et les alliages de ces métaux. Parmi ces métaux, on préfère tout particulièrement l'or, l'argent, le platine, le cadmium, le sélénium et les alliages de ces métaux.

Les particules d'un ou plusieurs composés minéraux peuvent être également des oxydes. On peut citer les oxydes des éléments des colonnes 1 à 14 du tableau de classification périodique des éléments. En particulier, on peut notamment citer les oxydes de titane, de zinc, de cérium, de zirconium, d'aluminium et d'oxychlorure de bismuth. Parmi ces composés, on préfère tout particulièrement l'oxyde de zinc.

Les particules d'un ou plusieurs composés minéraux peuvent être des sels inorganiques. On peut notamment citer le sulfate de baryum, le carbonate de calcium, le sulfate de calcium, le phosphate de calcium, l'hydrocarbonate de magnésium. Parmi ces composés, on préfère le carbonate de calcium.

Les particules d'un ou plusieurs composés minéraux peuvent être des carbures, des nitrures, des borures, des sulfures et des hydroxydes.

Parmi les particules d'un ou plusieurs composés minéraux appartenant aux espèces décrites ci-dessus, on peut aussi citer les argiles, les silicates, l'alumine, la silice, le kaolin et l'hydroxyapatite.

De préférence, les particules d'un ou plusieurs composés minéraux sont choisies parmi le kaolin, l'oxyde de zinc, le carbonate de calcium et l'argent.

Les particules d'un ou plusieurs composés minéraux présentent, de préférence, une taille primaire moyenne en nombre comprise entre 0,001 et 1000 µm, de préférence comprise entre 0,01 et 700 µm.

Au sens de la présente invention, on entend par « taille primaire de particule », la dimension maximale qu'il est possible de mesurer entre deux points diamétralement opposés d'une particule individuelle.

La taille des particules d'un ou plusieurs composés minéraux peut être déterminée par microscopie électronique à transmission ou à partir de la mesure de la surface spécifique par la méthode BET ou à partir d'une granulométrie laser.

La ou les particules d'un ou plusieurs composés minéraux utilisées dans la composition cosmétique peuvent présenter différentes formes, par exemple une forme de sphères, de paillettes, d'aiguilles ou de plaquettes et de préférence elles sont sensiblement sphériques.

La ou les particules d'un ou plusieurs composés minéraux utilisées dans la composition cosmétique selon l'invention est ou sont présentes dans une teneur allant de 0,01 à 25 % en poids, de préférence allant de 0,1 à 10% en poids, par rapport au poids total de la composition.

Par milieu cosmétiquement acceptable, on entend au sens de la présente invention, un milieu compatible avec les matières kératiniques, telles les cheveux et la peau.

Le milieu cosmétiquement acceptable est constitué d'eau ou d'un mélange d'eau et d'au moins un solvant cosmétiquement acceptable choisi parmi les alcools inférieurs en C₁-C₄, tels que l'éthanol, l'isopropanol, le tertio-butanol ou le n-butanol ; les polyols tels que le glycérol, le propylèneglycol et les polyéthylèneglycols ; et leurs mélanges.

Le pH des compositions selon l'invention est généralement inférieur à 7, de préférence inférieur à 6, mieux compris entre 2 et 6, et encore plus préférentiellement entre 3 et 6.

En particulier, les compositions obtenues sont stables à un pH inférieur à 6.

La composition selon l'invention peut comprendre en outre un ou plusieurs additifs classiques bien connus dans la technique, tels que; des épaississants ou régulateurs de viscosité, naturels ou synthétiques ; des alcools gras en C₁₂-C₃₀ ; des céramides ; des esters gras huileux tels que le myristate d'isopropyle ; des huiles minérales, végétales ou synthétiques telles que les α-oléfines ; des vitamines ou provitamines ; des agents nacrants ; des agents de stabilisation du pH, des conservateurs ; et des colorants.

L'homme de métier veillera à choisir les éventuels additifs et leur quantité de manière à ce qu'ils ne nuisent pas aux propriétés des compositions de la présente invention.

Ces additifs sont généralement présents dans la composition selon l'invention en une quantité allant de 0 à 20 % en poids par rapport au poids total de la composition.

Les compositions conformes à l'invention peuvent être utilisées pour le lavage et le conditionnement des matières kératiniques, en particulier des cheveux, par exemple comme shampoings conditionneurs,
ou encore pour le conditionnement des matières kératiniques, par exemple comme après-shampoings.

Un autre objet de l'invention est un procédé de traitement cosmétique des matières kératiniques, telles que les cheveux, qui consiste à appliquer une quantité efficace d'une composition telle que décrite ci-dessus, sur lesdites matières, à rincer après un éventuel temps de pose.

L'exemple suivant illustre la présente invention.

### EXEMPLE

On prépare les compositions A, B, C et D à partir des ingrédients indiqués dans le tableau ci-dessous.

Les quantités de matières premières sont indiquées en l'état.

| Compositions | A | B | C | D |
|---|---|---|---|---|
| Kaolin ⁽⁷⁾ | 0,5 | - | - | - |
| Oxyde de zinc ⁽⁸⁾ | - | 0,5 | - | - |
| Carbonate de calcium ⁽⁹⁾ | - | - | 0,5 | - |
| Argent ⁽¹⁰⁾ | - | - | - | 3 |
| Chlorure de sodium | 0,15 | 0,15 | - | - |
| Mélange de méthylparaben, de butylparaben, éthylparaben, isoparaben et propylparaben (Conservateurs) | 0,5 | 0,5 | 0,5 | 0,5 |
| Polyacrylate-1 Crosspolymer en émulsion à 20 % dans l'eau⁽¹⁾ | 5 | 5 | 5 | 5 |
| Polyquaternium-10 ⁽⁵⁾ | 0,8 | - | - | 0,5 |
| Polyéthylèneimine-10 (nom INCI PEI-10) ⁽⁴⁾ | - | 0,025 | 0,025 | - |
| Polyquaternium-6 ⁽⁶⁾ | - | 0,1 | 0,1 | - |
| Laureth-2 | 0,9 | 0,9 | 0,9 | 0,9 |
| Cocoyl amidopropyl bétaïne en solution aqueuse (38% MA) | 6,4 | 6,4 | 6,2 | 6,2 |
| Lauryl éther sulfate de sodium (2.2 OE) en solution aqueuse (72 %MA) ⁽³⁾ | 22,2 | 22,2 | 17,85 | 17,85 |
| Acide citrique qsp | pH=5,5 | pH=5,5 | pH=5,5 | pH = 5,5 |
| Eau désionisée | Qs 100 | Qs 100 | Qs 100 | Qs 100 |

| | | | | |
|---|---|---|---|---|
| ⁽¹⁾ vendu sous la dénomination commerciale Carbopol Aqua CC par la société Noveon ⁽²⁾ vendu sous la dénomination Tego Betaïne F50 par la société GOLDSHMIDT ⁽³⁾ vendu sous la dénomination Texapon AOS 225UP par la société COGNIS ⁽⁴⁾ vendu sous la dénomination Lupasol FG par la société BASF ⁽⁵⁾ vendu sous la dénomination JR400LT par la société AMERCHOL ⁽⁶⁾ vendu sous la dénomination Merquat 100 par la société NALCO ⁽⁷⁾ vendu sous la dénomination Kaolin Polwhite B par la société IMERYS ⁽⁸⁾ vendu sous la dénomination Oxyde de zinc micropur par la société LCW ⁽⁹⁾ vendu sous la dénomination Omyapure 35 par la société OMYA ⁽¹⁰⁾ vendu sous la dénomination Nanocrystalline Silver par la société Nanophases Technologies | | | | |

Ces compositions constituent des shampooings stables. Ils s'appliquent sur les cheveux sans couler. Après un rinçage aisé, ils confèrent aux cheveux de bonnes propriétés de démêlage, de douceur et de maintien.

## Revendications

1. composition de traitement cosmétique des matières kératiniques, de préférence humaines, en particulier des fibres kératiniques, et plus particulièrement des cheveux, comprenant, dans un milieu cosmétiquement acceptable :
- (i) un ou plusieurs polymères cationiques obtenus par polymérisation d'un mélange de monomères comprenant un ou plusieurs monomères vinyliques substitués par un ou plusieurs groupes amino, un ou plusieurs monomères vinyliques non ioniques hydrophobes, un ou plusieurs monomères vinyliques associatifs, et un ou plusieurs monomères vinyliques non ioniques hydroxylés ;
- (ii) un ou plusieurs agents tensioactifs,
- (iii) un ou plusieurs polymères cationiques ou amphotères différents des polymères (i),
- (iv) une ou plusieurs particules d'un ou plusieurs composés minéraux.

2. Composition selon la revendication 1, **caractérisée en ce que** le ou les monomères vinyliques substitués par un ou plusieurs groupes amino sont choisis parmi :
- les (méth)acrylates de mono(alkyl en C₁-C₄)amino(alkyle en C₁-C₈),
- les (méth)acrylates de di(alkyl en C₁-C₄)amino(alkyle en C₁-C₈), de préférence les (méth)acrylates de di(alkyl en C₁-C₄)alkylamino(alkyle en C₁-C₆),
- les mono(alkyl en C₁-C₄)amino(alkyl en C₁-C₈)(méth)acrylamides,
- les di(alkyl en C₁-C₄)amino(alkyl en C₁-C₈)(méth)acrylamides,
- les (méth)acrylamides à groupement hétérocyclique contenant un atome d'azote,
- les (méth)acrylates à groupement hétérocyclique contenant un atome d'azote,
- les hétérocycles azotés à groupement(s) vinyle(s),
- et leurs mélanges.

3. Composition selon l'une quelconque des revendications précédentes, **caractérisée en ce que** le ou les monomères vinyliques non ioniques hydrophobes répondent à la formule (I) ou (II) :
(I) CH₂=C(X)Z,
(II) CH₂=CH-OC(O)R;
dans lesquelles formules (I) et (II) :
X représente H ou un groupe méthyle ;
Z est choisi parmi les groupes -C(O)OR¹, -C(O)NH₂, -C(O)NHR¹, -C(O)N(R¹)₂, -C₆H₅, -C₆H₄R¹, -C₆H₄OR¹, -C₆H₄Cl, -CN, -NHC(O)CH₃, - NHC(O)H, N-(2-pyrrolidonyle), N-caprolactamyle, -C(O)NHC(CH₃)₃, - C(O)NHCH₂CH₂-NH-CH₂CH₂-urée, -SiR₃, -C(O)O(CH₂)ₓSiR₃, - C(O)NH(CH₂)ₓSiR₃ et -(CH₂)ₓSiR₃ ;
x représente un nombre entier allant de 1 à 6 ;
chaque R représente indépendamment un groupe alkyle en C₁-C₃₀ ;
chaque R¹ représente indépendamment un groupe alkyle en C₁-C₃₀ , un groupe alkyle en C₂-C₃₀ hydroxylé, ou un groupe alkyle en C₁-C₃₀ halogéné.

4. Composition selon l'une quelconque des revendications précédentes, **caractérisée en ce que** le ou les monomères vinyliques associatifs sont choisis parmi les composés de formule (III) : 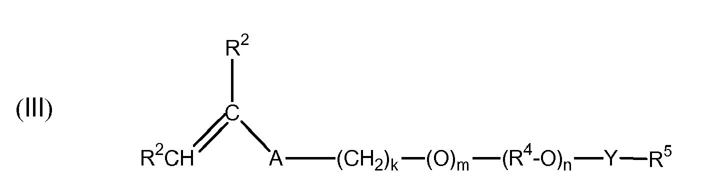 dans laquelle
chaque R² représente indépendamment H, un groupe méthyle, un groupe -C(O)OH, ou un groupe -C(O)OR³ ;
R³ représente un groupe alkyle en C₁-C₃₀ ;A représente un groupe -CH₂C(O)O-, -C(O)O-, -O-, -CH₂O-, -NHC(O)NH-, -C(O)NH-, -Ar-(CE₂)_{z}-NHC(O)O-,
-Ar-(CE₂)_{z}-NHC(O)NH- ou -CH₂CH₂-NHC(O)- ;
Ar représente un groupe arylène ;
E représente H ou un groupe méthyle ;
z est égal à 0 ou 1 ;
k est un nombre entier allant de 0 à 30 ;
m est égal à 0 ou 1, à la condition que lorsque k = 0, m = 0, et lorsque k varie de 1 à 30, m est égal à 1 ;
(R⁴-O)ₙ représente un groupement polyoxyalkylène, qui est un homopolymère, un copolymère aléatoire, ou un copolymère à blocs, comportant des motifs oxyalkylène en C₂-C₄,
R⁴ représente -C₂H₄-, -C₃H₆-, -C₄H₈- ou leurs mélanges,
n est un entier variant de 5 à 250,
Y rerpésente -R⁴O-, -R⁴NH-, -C(O)-, -C(O)NH-, R⁴NHC(O)NH-, ou -C(O)NHC(O)- ;
R⁵ représente un groupe alkyle substitué ou non, choisi parmi les groupes alkyles linéaires en C₈-C₄₀, les groupes alkyles ramifiés en C₈-C₄₀, les groupes alicycliques en C₈-C₄₀, les groupes phényles substitués par un groupe alkyle en C₂-C₄₀, les groupes alkyles en C₂-C₄₀ substitués par un groupe aryle, et les esters complexes en C₈-C₈₀, le groupe alkyle R⁵ comprenant éventuellement un ou plusieurs substituants choisis parmi les groupes hydroxyle, alcoxyle et halogéno.

5. Composition selon l'une quelconque des revendications précédentes, **caractérisée en ce que** le mélange de polymères comprend en outre un ou plusieurs monomères vinyliques non ioniques hydroxylés choisis parmi les (méth)acrylates d'hydroxyalkyle en C₁-C₆, les (hydroxyalkyl en C₁-C₄)(méth)acrylamides, et leurs mélanges.

6. Composition selon l'une quelconque des revendications précédentes, **caractérisée en ce que** le mélange de monomères comprend un ou plusieurs monomères tensioactifs vinyliques semi-hydrophobes.

7. Composition selon la revendication 6, **caractérisée en ce que** le ou les monomères tensioactifs vinyliques semi-hydrophobes sont choisis parmi les composés de formules (IV) ou (V) : 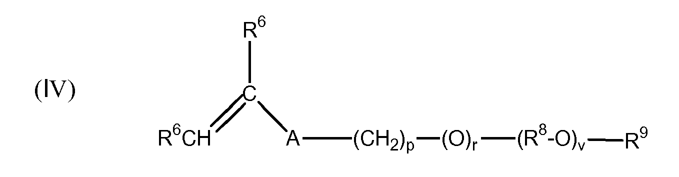 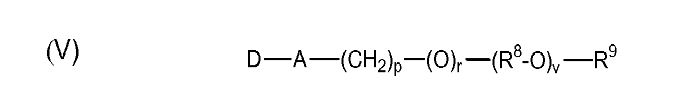 dans lesquelles formules (IV) et (V) :
chaque R⁶ représente indépendamment H, un groupe alkyle en C₁-C₃₀, -C(O)OH, ou -C(O)OR⁷ ;
R⁷ représente un groupe alkyle en C₁-C₃₀ ;
A représente un groupe -CH₂C(O)O-, -C(O)O-, -O-, -CH₂O-, -NHC(O)NH-, -C(O)NH-, -Ar-(CE₂)_{z}-NHC(O)O-,
-Ar-(CE₂)_{z}-NHC(O)NH- ou -CH₂CH₂NHC(O)- ;
Ar représente un groupe arylène ;
E représente H ou un groupe méthyle ;
z est égal à 0 ou 1 ;
p est un nombre entier allant de 0 à 30 ;
r est égal à 0 ou 1, à la condition que lorsque p est égal à 0, r est égal à 0, et lorsque p varie de 1 à 30, r est égal 1,
(R₈-O)ᵥ représente un groupement polyoxyalkylène qui est un homopolymère, un copolymère aléatoire, ou un copolymère à blocs avec des motifs oxyalkylène en C₂-C₄, où R⁸ représente -C₂H₄-, -C₃H₆-, - C₄H₈- ou leurs mélanges, et v est un nombre entier allant de 5 à 250 ;
R⁹ représente H ou un groupe alkyle en C₁-C₄ ; D représente un groupe alcényle en C₈-C₃₀ ou un groupe alcényle en C₈-C₃₀ substitué par un groupe carboxy.

8. Composition selon l'une quelconque des revendications précédentes, **caractérisée en ce que** le ou les polymères cationiques (i) est ou sont obtenus par polymérisation du mélange de monomères comprenant, par rapport au poids total du mélange de monomères :
a)de 10 à 70 % en poids d'un ou plusieurs monomères vinyliques substitués par un ou plusieurs groupes amino,
b)de 20 à 80 % en poids d'un ou plusieurs monomères vinyliques non ioniques hydrophobes,
c)de 0,001 à 25 % en poids d'un ou plusieurs monomères vinyliques associatifs,
d)de 0 à 25 % en poids d'un ou plusieurs monomères tensioactifs vinyliques semi-hydrophobes,
e)de 0 à 10 % en poids d'un ou plusieurs monomères vinyliques non ioniques hydroxylés,
f) de 0 à 5% en poids d'un ou plusieurs monomères réticulants,
g)de 0 à 10 % en poids d'un ou plusieurs agents de transfert de chaîne, et
h)de 0 à 2 % en poids d'un ou plusieurs agents de stabilisation polymérique.

9. Composition selon l'une quelconque des revendications précédentes, **caractérisée en ce que** le ou les polymères cationiques (i) est ou sont obtenus par polymérisation du mélange de monomères comprenant, par rapport au poids total du mélange de monomères :
a) de 20 à 60 % en poids du ou des monomères vinyliques substitués par un ou plusieurs groupes amino,
b) de 20 à 70 % en poids du ou des monomères vinyliques non ioniques hydrophobes,
c) de 0,01 à 15 % en poids du ou des monomères vinyliques associatifs,
d) de 0,1 à 10 % en poids du ou des monomères tensioactifs vinyliques semi-hydrophobes,
e) de 0,01 à 10 % en poids du ou des monomères vinyliques non ioniques hydroxylés,
f) de 0,001 à 5 % en poids du ou des monomères réticulants,
g) de 0,001 à 10 % en poids du ou des agents de transfert de chaîne, et
h) de 0 à 2 % en poids du ou des agents de stabilisation polymériques.

10. Composition selon l'une quelconque des revendications précédentes, **caractérisée en ce que** le polymère cationique (i) est obtenu par polymérisation du mélange de monomères suivants :
- un méthacrylate de di(alkyl en C₁-C₄)amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé avec 20 à 30 moles d'oxyde d'éthylène,
- un allyl éther de polyéthylèneglycol/polypropylèneglycol 30/5,
- un méthacrylate d'hydroxy(alkyle en C₂-C₆),
- un diméthacrylate d'éthylèneglycol.

11. Composition selon l'une quelconque des revendications précédentes, **caractérisée en ce qu'**elle comprend un ou plusieurs tensioactifs anioniques et un ou plusieurs tensioactifs amphotères ou zwittérioniques.

12. Composition selon la revendication 11, **caractérisée en ce que** le tensioactif anionique est choisi parmi les alkylsulfates, les alkyléthersulfates et leurs mélanges.

13. Composition selon la revendication 11, **caractérisée en ce que** le tensioactif amphotère ou zwittérionique est choisi parmi les (alkyl en C₈₋₂₀)-bétaïnes, les (alkyl en C₈₋₂₀)-amido(alkyl en C₆₋₈)bétaïnes et leurs mélanges.

14. Composition selon l'une quelconque des revendications précédentes, **caractérisée en ce que** le ou les polymères cationiques (iii) sont choisis parmi les dérivés d'éther de cellulose comportant des groupements ammonium quaternaires, les cyclopolymères cationiques, les gommes de guar modifiées par un sel de 2,3-époxypropyl-triméthylammonium, les polymères quaternaires de vinylpyrrolidone et de vinylimidazole, et les homopolymères réticulés de sels de méthacryloyloxyalkyl(C₁-C₄) trialkyl(C₁-C₄)ammonium.

15. Composition selon l'une quelconque des revendications précédentes, **caractérisée en ce que** la ou les particules d'un ou plusieurs composés minéraux sont choisies les particules métalliques, les oxydes, les sels inorganiques, les carbures, les nitrures, les sulfures et les hydroxydes.

16. Procédé de traitement cosmétique des matières kératiniques, **caractérisé en ce que** l'on applique une quantité efficace d'une composition selon l'une quelconque des revendications précédentes, sur lesdites matières, à rincer après un éventuel temps de pose.

17. Utilisation d'une composition selon l'une quelconque des revendications 1 à 15, comme shampoing conditionneur ou comme après shampooing.
